# EUROPEAN PATENT APPLICATION

(11) **EP 4 703 464 A1**
(43) Date of publication of application: **04.03.2026**
(21) Application number: 24797202.9
(22) Date of filing: 26.04.2024
(51) Int. Cl.: C12N 5/071

(54) **METHOD FOR PRODUCING THREE-DIMENSIONAL CELL STRUCTURE**

(30) Priority: 28.04.2023 JP 2023074637
(71) Applicant: Tissuebynet Corporation, Saitama 355-0344 (JP); Kyoto University, Kyoto-shi, Kyoto 606-8501 (JP); National Cerebral and Cardiovascular Center, Suita-shi, Osaka 564-8565 (JP)
(72) Inventor: MORIMOTO Naoki, Kyoto-shi, Kyoto 606-8501 (JP); YAMAOKA Tetsuji, Kishibe-Shimmachi, Suita-shi, Osaka 564-8565 (JP); SAKAMOTO Michiharu, Kyoto-shi, Kyoto 606-8501 (JP); NAKANO Takashi, Kyoto-shi, Kyoto 606-8501 (JP); ONO Jiro, Hiki-gun, Saitama 355-0344 (JP); NAKAHASHI Kyoko, Kishibe-Shimmachi, Suita-shi, Osaka 564-8565 (JP)
(74) Representative: Müller-Boré & Partner Patentanwälte PartG mbB
(86) International application number: PCT/JP2024/016528
(87) International publication number: WO 2024/225459

(57) **Abstract**

The purpose of the present invention is to provide a three-dimensional cell structure having strength that enables suturing. This method for producing a three-dimensional cell structure includes: (1) a step for preparing original cells having proliferation capability, and a culture chamber which includes a cavity having an internal space surrounded by a lower surface, an upper surface, and side surfaces, and which is formed such that the culture liquid can circulate in the internal space; (2) a step for supplying the original cells to the culture chamber; (3) a step for holding the whole culture chamber in a culture liquid; and (4) a step for culturing the original cells, integrating the original cells, the cultured cells derived therefrom, and an extracellular matrix produced during the culturing, and forming a three-dimensional cell structure that has grown to the outside of the cavity.

## Description

### Technical Field

The present invention relates to a method for producing a three-dimensional cell structure, and particularly relates to a method for producing a high-strength three-dimensional cell structure, a three-dimensional cell structure produced by the method, a method for producing artificial skin using the same, and artificial skin produced by the method.

### Background Art

In recent years, regenerative medicine using cultured cells has been attracting attention as a new medical technology. The production of three-dimensional cell structures such as artificial tissues and artificial organs for the purpose of transplantation into the human body is being researched. Artificial skin is a field that is relatively easy to enter in the field of regenerative medicine for reasons such as being relatively easy to access for application to the surface of the human body, allowing the post-transplantation situation to be visually confirmed, and enabling re-operation of only a defective part if one exists. Three-dimensional cell structures such as artificial tissues and artificial organs can also be used for pharmacological tests, toxicity tests, embryological tests, or the like. If a three-dimensional cell structure can be produced using human cells or the like, and tests can be easily conducted in an environment close to the in vivo environment, it is expected that drug discovery research, personalized medication diagnosis, observational research on the development of various organs, or the like will become more efficient.

Patent Literature 1 and Non-Patent Literature 1 describe a method for producing a three-dimensional cell structure by culturing and fusing a plurality of cell aggregates (spheroids) in a space within a support body formed by a plurality of thread-like or needle-like members. Approved products in the field of clinically used artificial skin include human (autologous) epidermis-derived cell sheets and bilayer artificial skin composed of a collagen sponge layer and a reinforcing film.

### Citation List

### Patent Literatures

Patent Literature 1: International Publication No. WO2018/051415

### Non Patent Literatures

Non Patent Literature 1: Bioengineering of a scaffold-less three-dimensional tissue using net mould, Katsuhisa Sakaguchi et al 2021 Biofabrication 13 045019, https://doi.org/10.1088/1758-5090/ac23e3

### Summary of Invention

### Problems to be solved by the invention

Three-dimensional cell structures produced by conventional methods and epidermis-derived cell sheets and artificial skin used clinically do not have a strength capable of being sutured, and their range of use has been limited. An object of the present invention is to provide a three-dimensional cell structure having a strength capable of being sutured.

### Means for solution of the problems

The present inventors, as a result of diligent studies to solve the above problem, have found that it is possible to produce a three-dimensional cell structure having a strength capable of being sutured by three-dimensionally culturing original cells including fibroblasts and/or cells having fibroblast differentiation potential using a specific method, thereby completing the present invention. That is, the present invention provides a method for producing a three-dimensional cell structure, a method for producing artificial skin, a three-dimensional cell structure, and artificial skin, as shown below.
[1] A method for producing a three-dimensional cell structure, comprising:
   (1) a step of preparing original cells having proliferative ability, and a culture chamber that includes a cavity having an internal space enclosed by a bottom surface, a top surface, and side surfaces, and that is formed such that a culture medium can circulate within the internal space;
   (2) a step of supplying the original cells to the culture chamber;
   (3) a step of holding the entire culture chamber in a culture medium; and
   (4) a step of culturing the original cells to form a three-dimensional cell structure that is configured by the original cells, cultured cells derived therefrom, and an extracellular matrix produced during culture becoming integrated, and that has grown to outside the cavity.
[2] The method according to [1], wherein the original cells include fibroblasts and/or cells having fibroblast differentiation potential.
[3] The method according to [1], wherein the original cells are in the form of spheroids.
[4] The method according to [1], wherein step 2 includes a step of filling the internal space of the cavity with the original cells.
[5] The method according to [1], wherein the original cells are cultured until the breaking force of the three-dimensional cell structure becomes 0.08 N or more.
[6] The method according to [1], further comprising a step of collecting a portion of the three-dimensional cell structure that has overflowed to the outside of the cavity.
[7] The method according to [1], wherein the three-dimensional cell structure includes a cultured dermis.
[8] The method according to [1], wherein the bottom surface and/or the top surface of the cavity has a comb-like shape or a net-like shape.
[9] The method according to [1], wherein the side surfaces of the cavity are formed by laminating a plurality of frames.
[10] A method for producing artificial skin, comprising a step of laminating a three-dimensional cell structure produced by the method according to any one of [1] to [9] with a member for an epidermis.
[11] A three-dimensional cell structure that includes original cells used at the start of culture therein, and that is configured by the original cells, cultured cells derived therefrom, and an extracellular matrix produced during culture becoming integrated, wherein the breaking force is 0.08 N or more.
[12] The three-dimensional cell structure according to [11], wherein the original cells include fibroblasts and/or cells having fibroblast differentiation potential.
[13] The three-dimensional cell structure according to [11], including a cultured dermis.
[14] Artificial skin, comprising the three-dimensional cell structure according to any one of [11] to [13] and a member for an epidermis laminated thereon.

### Advantageous Effects of Invention

According to the present invention, by growing a three-dimensional cell structure until it overflows from a cavity of a culture chamber, a three-dimensional cell structure having a strength capable of being sutured can be produced. Therefore, it is possible to provide a three-dimensional cell structure that is useful for transplantation into a living body.

### Brief Description of Drawings

Fig. 1 shows a schematic diagram of a cavity of a culture chamber in which a top surface, a bottom surface, and side surfaces are formed by wires, and a state where original cells are supplied thereto and the entire culture chamber is held in a culture medium, as one aspect of the present invention.
Fig. 2 shows schematic diagrams, as one aspect of the present invention, of (a) when the internal space of the cavity of the culture chamber is filled with original cells, (b) when a three-dimensional cell structure is formed within the cavity, and (c) when the three-dimensional cell structure has grown until it overflows from the cavity.
Fig. 3A shows a stained photograph (HE staining) of a cross-section of a three-dimensional cell structure produced as one aspect of the present invention.
Fig. 3B shows a stained photograph (EVG staining) of a cross-section of a three-dimensional cell structure produced as one aspect of the present invention.
Fig. 3C shows a stained photograph (Alcian blue staining) of a cross-section of a three-dimensional cell structure produced as one aspect of the present invention.
Fig. 3D shows a stained photograph (staining with an anti-type I collagen antibody) of a cross-section of a three-dimensional cell structure produced as one aspect of the present invention.
Fig. 3E shows a stained photograph (staining with an anti-type II collagen antibody) of a cross-section of a three-dimensional cell structure produced as one aspect of the present invention.
Fig. 4 shows external appearance photographs of a wound site in a transplantation experiment.
Fig. 5 shows a stained photograph (HE staining) of a section of a wound site in a transplantation experiment. The portion enclosed by a dashed line is the transplanted three-dimensional cell structure (cultured dermis).
Fig. 6 shows (a) an external appearance photograph of when original cells in the form of spheroids of a size that does not fit into a cavity of a culture chamber are supplied to the culture chamber, and (b) an external appearance photograph of a three-dimensional cell structure formed by culturing for one month thereafter.
Fig. 7 shows a stained photograph (HE staining) of a cross-section of a three-dimensional cell structure produced as one aspect of the present invention, viewed along line V-V in Fig. 6b, and a partially enlarged photograph thereof.

### Description of Embodiments

Hereinafter, the present invention will be described in further detail.

The present invention relates to a method for producing a three-dimensional cell structure, and includes a step (Step 1) of preparing original cells (original cells) having proliferative ability, and a culture chamber that includes a cavity having an internal space enclosed by a bottom surface, a top surface, and side surfaces, and that is formed such that a culture medium can circulate within the internal space. The "three-dimensional cell structure" described in this specification refers to a three-dimensional structure formed by multiple overlapping layers of cells, and refers to a cultured tissue that also includes an extracellular matrix.

The "original cells" described in this specification refer to the cells targeted for culture that are used at the start of culturing. The original cells are not particularly limited as long as they are capable of proliferating in vitro, and may include, for example, fibroblasts and/or cells having fibroblast differentiation potential, or embryonic stem cells or induced pluripotent stem cells. The original cells may be prepared as individual, separate cells, or may be prepared in the form of spheroids in which cells are aggregated. The size of the spheroids is not particularly limited, but for example, their particle size may be from about 100 to about 300 µm, from about 100 to 500 µm, from about 100 to about 750 µm, from about 100 to about 1000 µm, or from about 100 to about 2500 µm.

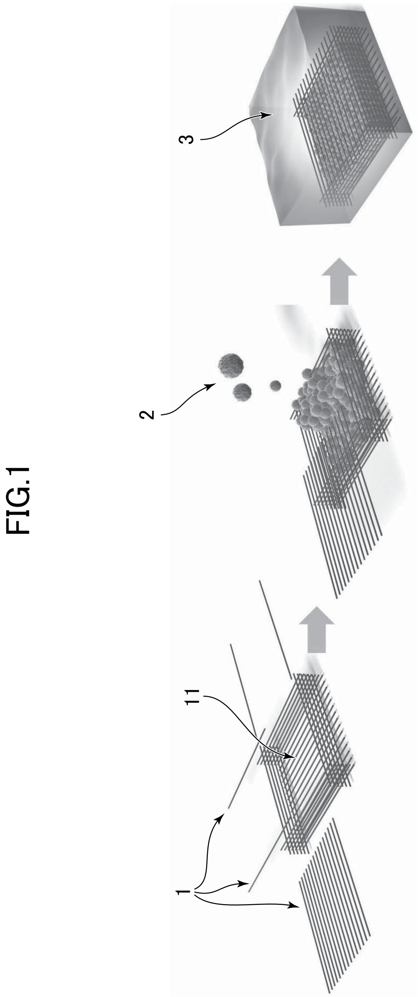

In the culture chamber, since it is necessary for the culture medium to be able to circulate within the internal space of the cavity, the culture chamber and the bottom surface, top surface, or side surfaces of the cavity have voids through which the culture medium can pass. The method of forming the voids is not particularly limited, but for example, the bottom surface and/or the top surface of the cavity may have a comb-like shape or a net-like shape, and the side surfaces of the cavity may be formed by laminating a plurality of frames, and voids may exist between the frames. Specifically, as shown in Fig. 1, the bottom surface, top surface, and side surfaces of the cavity of the culture chamber may be configured with a plurality of wires 1 arranged in a comb-like shape. The culture medium can freely pass through the voids between the wires and circulate within the internal space 11 of the cavity of the culture chamber. More specifically, the culture chamber described in Patent Literature 1 or Non-Patent Literature 1, or a commercially available net mold (produced by Tissue By Net Inc.) may be used.

The method of the present invention includes a step (Step 2) of supplying the original cells to the culture chamber. In this step, the original cells may be supplied into the internal space of the cavity of the culture chamber, or may be placed on top of the cavity and then pushed into the internal space of the cavity when the top surface of the culture chamber is set.

In one aspect, Step 2 includes a step of filling the internal space of the cavity with the original cells. "Filling" herein refers to a state where the original cells have been introduced until they are in contact with the top surface of the cavity, and also includes the case where, as a result of placing the original cells on top of the cavity and pushing them in when setting the top surface of the culture chamber, a state of being introduced until they are in contact with the top surface of the cavity is achieved.

The method of the present invention includes a step (Step 3) of holding the entire culture chamber in a culture medium. In the culture chamber, since the culture medium can circulate within the internal space of the cavity, for example, as shown in Fig. 1, if the entire culture chamber is held in the culture medium 3 after supplying the original cells 2 into the internal space 11 of the cavity of the culture chamber, the culture medium will be distributed to the original cells supplied into the internal space of the cavity. The composition of the culture medium is not particularly limited, and can be appropriately selected according to the type of original cells and the purpose of the culture.

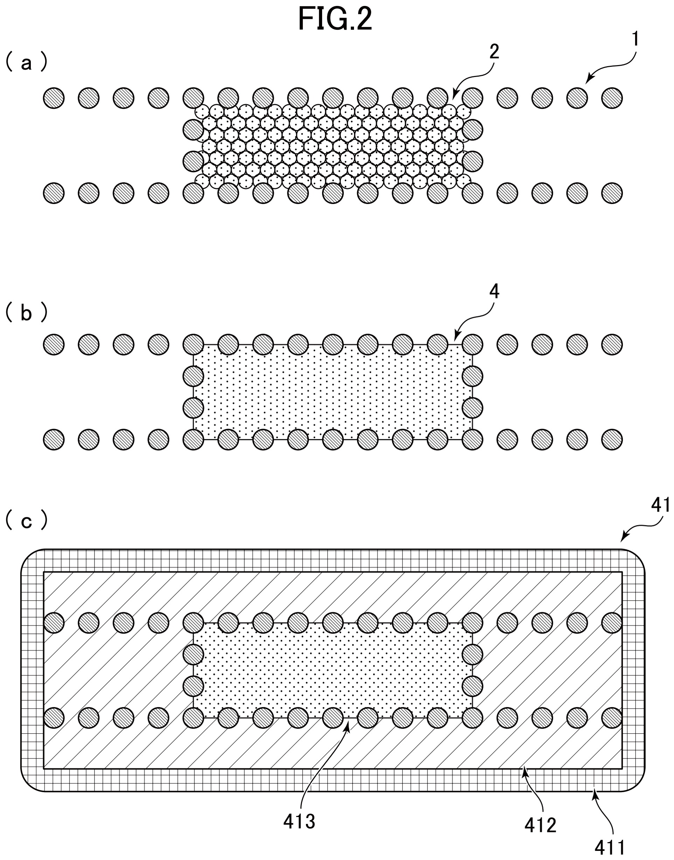

The method of the present invention includes a step (Step 4) of culturing the original cells to form a three-dimensional cell structure that is configured by the original cells, cultured cells derived therefrom, and an extracellular matrix produced during culture becoming integrated, and that has grown to outside the cavity. That is, in the method of the present invention, culturing is continued until the formed three-dimensional cell structure grows beyond any of the top surface, bottom surface, or side surfaces of the cavity and overflows to the outside thereof. For example, as shown in Fig. 2, culturing is started by filling the internal space of a cavity whose top surface, bottom surface, and side surfaces are configured with a plurality of wires 1 with original cells 2 (Fig. 2a), culturing is continued even after a three-dimensional cell structure 4 is formed in the internal space of the cavity (Fig. 2b), and the three-dimensional cell structure 41 is grown until it overflows from the cavity (Fig. 2c). The three-dimensional cell structure 41 that has grown until it overflows from the cavity is broadly configured from three parts, namely, a central portion 413 within the cavity containing many original cells 2, an outermost layer 411, and an intermediate layer 412 (Fig. 2c).

In one aspect, a portion of a three-dimensional cell structure that has overflowed from the side surface or the like of one cavity may become integrated with a portion of another three-dimensional cell structure that has overflowed from the side surface or the like of a nearby cavity to form a large three-dimensional cell structure.

In conventional culture methods, the objective was to form a three-dimensional cell structure configured by original cells, cultured cells derived therefrom, and an extracellular matrix produced during culture becoming integrated, by supporting it within the internal space of the cavity, and it was not envisioned that the three-dimensional cell structure would grow outside the cavity. However, surprisingly, it was found that by continuing to culture even after the three-dimensional cell structure was formed within the internal space of the cavity and by growing the three-dimensional cell structure to outside the cavity, its breaking force (breaking strength) increased, and a three-dimensional cell structure that can withstand suturing is formed.

The culture period of the original cells until the three-dimensional cell structure is formed in Step 4 is not particularly limited, but for example, when producing a three-dimensional cell structure in a single cavity, the period from the start of culture with the internal space of the cavity filled with the original cells may be about 2 months or more, about 3 months or more, about 4 months or more, or about 5 months or more, and may be about 10 months or less or about 9 months or less. Alternatively, when starting culture in a state where original cells in the form of spheroids larger than the internal space of a cavity in a culture chamber are placed at equal intervals on the cavity, and the spheroids are pushed into the internal space of the cavity by setting the top surface of the culture chamber, the culture period, although depending on the proportion of spheroids (original cells) occupying the entire culture chamber, may be, for example, about 2 weeks or more or about 1 month or more, and may be about 8 months or less or about 7 months or less. In one aspect, the original cells may be cultured until the breaking force of the three-dimensional cell structure becomes about 0.08 N or more, about 0.1 N or more, about 0.2 N or more, or about 0.3 N or more. The breaking force of the three-dimensional cell structure can be measured with a known breaking force measuring device or tensile testing machine such as an EZ Test (Shimadzu Corporation).

The three-dimensional cell structure produced by the method of the present invention has a strength capable of being sutured, and therefore can be advantageously used for transplantation into a living body. Its method of use is not particularly limited, and for example, the three-dimensional cell structure may be transplanted as a cultured dermis to a skin defect site such as a wound site, or the three-dimensional cell structure may be transplanted to a visceral defect site. Without being bound by any particular theory, for example, since the three-dimensional cell structure approximates the cell tissue structure of a living body, it is thought to function as a scaffold at the transplantation site, allowing cells from around the transplantation site to enter the inside of the three-dimensional cell structure and promote the regeneration of the skin or organ at the defect site. The three-dimensional cell structure is horizontally divisible into upper and lower parts centered on the central portion containing original cells, and in the three-dimensional cell structure divided into upper and lower parts, since the intermediate layer portion containing many non-stratified cells can be brought into contact with the transplant recipient surface, cells from around the transplantation site can easily enter, and a function as a scaffold can be better expected.

In one aspect, the method of the present invention may further include a step of collecting the portion of the three-dimensional cell structure that has overflowed to the outside of the cavity. If, after collecting this portion, culturing of the remaining portion including the central portion of the three-dimensional cell structure is continued, the three-dimensional cell structure can grow again to outside the cavity, and that overflowed portion can be collected again and used.

The method of the present invention may further include any arbitrary steps as long as the object of the present invention is not impaired. For example, the method of the present invention may further include a step of removing the formed three-dimensional cell structure from the culture chamber, and may include, during Step 4, a step of exchanging the culture medium and/or a step of washing the cells being cultured.

In another aspect, the present invention also relates to a three-dimensional cell structure that includes original cells used at the start of culture therein, and that is configured by the original cells, cultured cells derived therefrom, and an extracellular matrix produced during culture becoming integrated, wherein the breaking force of the three-dimensional cell structure is about 0.08 N or more, about 0.1 N or more, about 0.2 N or more, or about 0.3 N or more. The three-dimensional cell structure of the present invention can be prepared according to the method for producing a three-dimensional cell structure described above as one aspect of the present invention.

In yet another aspect, the present invention also relates to a method for producing artificial skin, comprising a step of laminating a three-dimensional cell structure produced by the method of the present invention described above as one aspect thereof with a member for an epidermis. Alternatively, the present invention also relates to artificial skin, comprising a three-dimensional cell structure described above as one aspect of the present invention and an epidermal member laminated thereon. The artificial skin is useful for transplantation to a wound site and the like.

As the member for an epidermis, those used in the art can be adopted without particular limitation, and for example, the member for an epidermis may be an epidermal cell sheet or the like. By laminating the three-dimensional cell structure and the member for an epidermis, the engraftment efficiency when the artificial skin is transplanted to a living body is increased.

Hereinafter, the present invention will be specifically described by way of Examples, but the scope of the present invention is not limited to these Examples. Examples

### [Test Example 1] Preparation of Three-dimensional Cell Structure

### (1) Cell Culture

Normal human dermal fibroblasts (NHDF; CC-2509, Lonza) were cultured for 3 days in an incubator (37°C, 5% CO₂) in FKCM medium (Fukoku Co., Ltd.) supplemented with 10% fetal bovine serum (Gibco FBS, Thermo Fisher Scientific) and 1% penicillin-streptomycin (Sigma-Aldrich). The proliferated cells were passaged, and cells cultured up to 4 to 5 passages were used in the subsequent tests.

### (2) Preparation of Spheroids

Spheroids were prepared with a silicon dimple plate (DP10-1, Tissue By Net Inc.). Specifically, NHDF were collected using TrypLE Express (Thermo Fisher Scientific), and a cell suspension of 1×10⁶ cells/mL was prepared using FKCM medium. 20 mL of the cell suspension was seeded onto a silicon dimple plate (DP10-1, Tissue By Net Inc.) having 4500 dimples per plate, and cultured for 12 hours in an incubator (37°C, 5% CO₂). As a result, spheroids with a diameter of about 200 µm were obtained.

### (3) Preparation of Three-dimensional Cell Structure

On the top surface and the bottom surface of a Net Mold NM14-2 (Tissue By Net Inc.), stainless steel wires with a diameter of 100 µm are arranged in parallel (in a comb-like shape) at 100 µm intervals, and are configured such that spheroids cannot pass through. In this test, a Net Mold NM14-2 was used, and by stacking ten 0.1 mm-thick side nets, a culture chamber including a cavity having an internal space of 6 mm × 6 mm × 1 mm was formed. The above spheroids were supplied into this internal space by a conventional method, and this culture chamber was placed in a 120 mL capacity sterile container (Thermo Fisher Scientific) containing 40 mL of FKCM medium (supplemented with 10% fetal bovine serum and 1% penicillin-streptomycin).

The sterile container including the culture chamber was set on a high-humidity compatible shaker CS-LR (TAITEC CORPORATION) in an incubator (37°C, 5% CO₂), and was shaken at 40 rpm to promote circulation of the medium. In this manner, the spheroids were cultured for 2 to 6 months while exchanging half the volume of the medium every week. When the culture period reached 2 months, the formed three-dimensional cell structure overflowed by about 500 µm from the top surface and the bottom surface of the cavity, respectively, and overflowed by about several millimeters from the side surfaces of the cavity, but even with further culturing, the thickness of the portions that overflowed from the top surface and the bottom surface did not change much.

After culturing, the three-dimensional cell structure in the Net Mold was removed by a conventional method and used in the subsequent tests. A sample with a diameter of 3 mm was prepared by cutting it out with a 3 mm diameter biopsy punch (Kai Industries Co., Ltd.).

### (4) Tissue Staining

The three-dimensional cell structure (cultured for 6 months) cut out to a diameter of 3 mm was fixed with formalin and embedded in paraffin by a conventional method, and a 5 µm-thick section was prepared at the center of the three-dimensional cell structure. This section was subjected to hematoxylin-eosin (HE) staining, Elastica van Gieson (EVG) staining, or Alcian blue staining, or was immunostained by a conventional method using an anti-type I collagen antibody or an anti-type II collagen antibody.

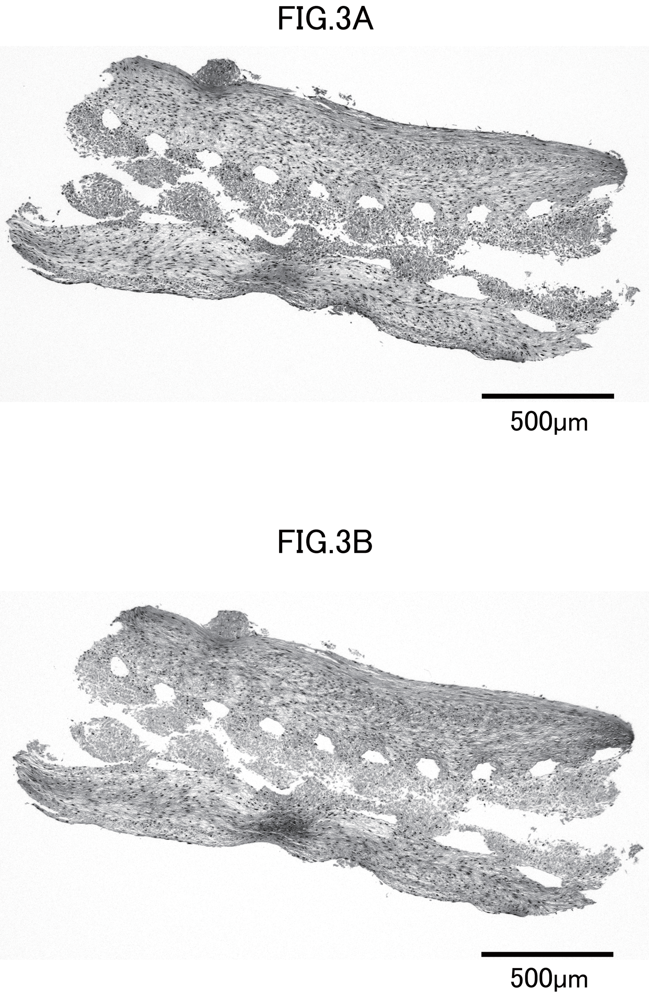

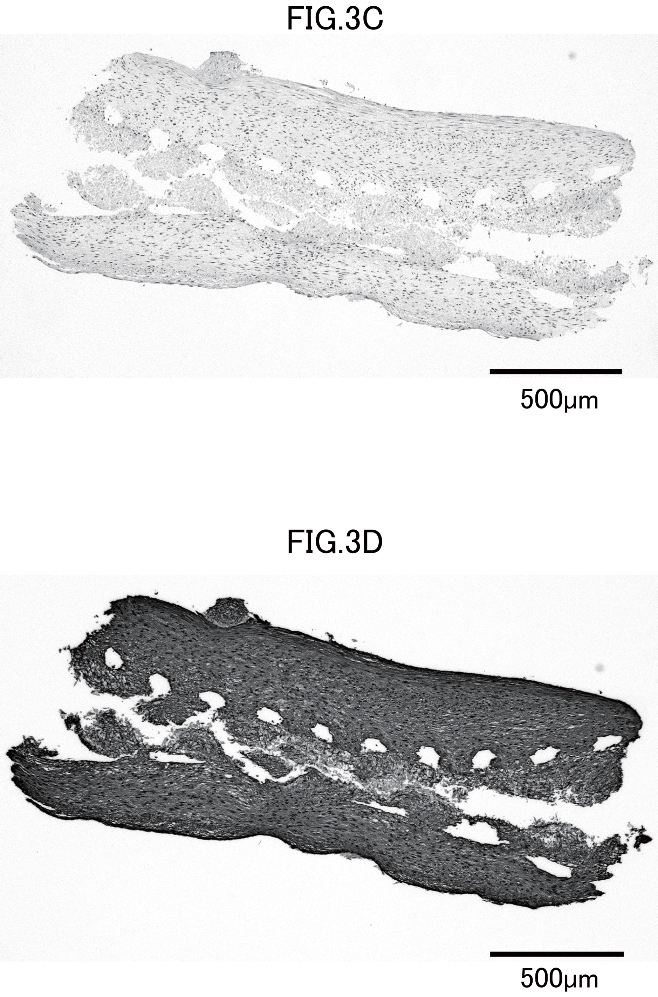

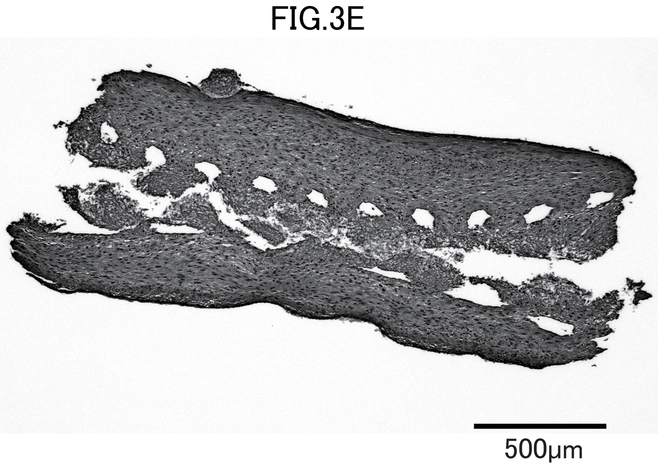

Looking at the optical micrograph of HE staining (Fig. 3A), it was found that outside the central portion where the spheroids existed at the start of culture, an intermediate layer with a relatively low cell density but constituting a tissue via an extracellular matrix, and an outermost layer containing a dense multilayer structure on the surface of the three-dimensional cell structure were formed. According to the optical micrograph of EVG staining (Fig. 3B), a three-dimensional cell structure mainly composed of collagen fibers was formed. According to the optical micrograph of Alcian blue staining (Fig. 3C), it was found that acid mucopolysaccharides were abundant in the intermediate layer portion of the three-dimensional cell structure. Then, according to the optical micrographs of immunostaining with the anti-type I collagen antibody or the anti-type II collagen antibody (Fig. 3D and Fig. 3E), it was found that type I collagen and type II collagen were widely present throughout the entire three-dimensional cell structure, and were particularly abundant in the outermost layer.

From these tissue staining results, it can be understood that the three-dimensional cell structure is configured by the original cells used at the start of culture, cultured cells derived therefrom, and an extracellular matrix such as acid mucopolysaccharides and collagen becoming integrated, and, since the three-dimensional cell structure has a structure similar to a living body's cell tissue structure (e.g., dermal structure), good compatibility upon transplantation into a living body was expected.

### [Test Example 2] Characterization of Three-dimensional Cell Structure

### (1) Measurement of Breaking Force

The three-dimensional cell structure was cut into a 6 mm square size, a polypropylene suture (thread size 5-0) was passed through its central part 2.5 mm from the bottom, and the ends of the thread were tied so that the length of the thread became 5 cm. The upper 3 mm portion of this was clamped by the upper clamp of a breaking force measuring device (EZ Test, Shimadzu Corporation), the lower end of the tied thread was clamped by the lower clamp, and it was pulled at a speed of 100 mm/min until the cultured dermis broke.

### (2) Measurement of Dry Weight

A 6 mm three-dimensional cell structure was dried for 3 days using a freeze dryer VD-250R (TAITEC CORPORATION), and its dry weight was measured with an electronic balance.

### (3) Measurement of Collagen Amount

Using a Collagen Quantitation Assay Kit (QuickZyme Biosciences), the amount of collagen in a 3 mm diameter three-dimensional cell structure sample was measured. The operation was performed according to the instruction manual, the absorbance of the reaction reagent at 570 nm was measured with a microplate reader, and the amount of collagen was calculated.

### (4) Measurement of Sulfated Glycosaminoglycan Amount

Using a Blyscan Glycosaminoglycan Assay Kit (Biocolor), the amount of sulfated glycosaminoglycan (sGAG) in a 3 mm diameter three-dimensional cell structure sample was measured. The operation was performed according to the instruction manual, the absorbance of the reaction reagent at 656 nm was measured with a microplate reader, and the amount of sGAG was calculated.

### (5) Comparison of Live Cell Count

Using a WST-8 based live cell counting reagent, Cell Count Reagent SF (Nacalai Tesque, Inc.), the number of live cells in a 3 mm diameter three-dimensional cell structure sample was measured. The operation was performed according to the instruction manual, the absorbance at a test wavelength of 450 nm and a reference wavelength of 650 nm was measured with a microplate reader, the difference in their absorbances was used as an indicator of the number of live cells, and the survival state of cells in the three-dimensional cell structure was compared.

### (6) Test Results

Table 1 shows the breaking force, dry weight, collagen amount, and sGAG amount measured using cultured dermis prepared by culturing for 2, 4, or 6 months.

**Table 1: Characteristics of Cultured Dermis (n=3)**

| Culture Period | Breaking Force (mN) | Dry Weight (mg) | Collagen Amount (µg) | sGAG Amount (µg) | Survival State ^{*1} |
|---|---|---|---|---|---|
| 2 months | 89±37 | 5.17±0.30 | 216.3±13.6 | 7.04±0.57 | 0.698±0.039 |
| 4 months | 520±54 | 4.95±0.19 | 171.1±14.1 | 6.07±0.42 | 0.534±0.079 |
| 6 months | 510±43 | 5.32±0.11 | 140.7±15.3 | 4.24±1.14 | 0.559±0.100 |

| | | | | | |
|---|---|---|---|---|---|
| ^{*1} OD450nm-OD650nm | | | | | |

Conventionally, the period for culturing spheroids using a Net Mold was about 3 weeks, but when spheroids were cultured beyond that, particularly from around after 2 months, the breaking force of the three-dimensional cell structure increased remarkably. On the other hand, there was no large difference in the dry weight of each three-dimensional cell structure, and the survival state of the cells was maintained in a good state despite the long-term culture. The collagen amount and the sGAG amount had a tendency to decrease according to the culture period.

For comparison, when the breaking force of a commercially available artificial skin composed of a collagen sponge layer (PELNAC Gplua^{(R)}, Gunze Ltd.) was measured, it was about 70 mN. The breaking force of the three-dimensional cell structure with a culture period of 2 months or more was higher than that, and was considered to be able to withstand suturing.

### [Test Example 3] Transplantation Experiment

### (1) Experimental Design and Surgical Method

A three-dimensional cell structure (cultured dermis) produced by culturing for 4 months by the method described in Test Example 1 was cut out with a 6 mm diameter biopsy punch (Kai Industries Co., Ltd.), and was divided horizontally into two upper and lower three-dimensional cell structures using forceps for use in animal experiments. Twenty-four BALB/cAJcl-nu/nu mice (male, 7 weeks old) (CLEA Japan, Inc.) were housed by a conventional method and were divided into three groups: a control group, a cultured dermis group, and a commercially available artificial dermis group. Note that all procedures involving pain were performed under isoflurane anesthesia.

In the application surgery, a donut-shaped silicone skin splint (outer diameter 18 mm, inner diameter 12 mm, thickness 0.5 mm; Fuji Systems Corporation) was attached to the skin with an adhesive (Aron Alpha; Daiichi Sankyo Company, Limited). The skin splint was sutured to the skin using 5-0 nylon thread (Bear Medic Corporation) to prevent contraction of the wound site. Then, in the inner center of the hole of the skin splint, a 6 mm diameter full-thickness skin defect wound was created using a 6 mm diameter biopsy punch (Kai Industries Co., Ltd.) and scissors.

Into the skin defect sites of the mice in the cultured dermis group and the commercially available artificial dermis group, cultured dermis for transplantation or PELNAC Gplus^{(R)} (Gunze Ltd.) was transplanted, respectively, and nothing was embedded in the skin defect sites of the mice in the control group. The wound site was covered with a silicone mesh sheet (diameter 6 mm, SI-mesh, ALCARE Co., Ltd.), and it was sutured to the epithelium at the edge with 5-0 nylon thread. Then, to prevent contamination and mechanical stress, the wound site was further covered with gauze and fixed with surgical tape (Silkytex, ALCARE Co., Ltd.). The mice after the procedure were placed in individual cages and housed in an animal facility.

### (2) Evaluation of Wound Healing

On the 7th and 14th postoperative days, the wound healing state was evaluated. At each evaluation time point, four mice from each group were sacrificed by carbon dioxide gas inhalation, and macroscopic observation images of the wound sites were acquired with a digital camera. Also, samples of the wound sites were collected together with the surrounding tissue, fixed with 10% formalin solution, embedded in paraffin, and each wound site was sectioned axially at the center. Then, the sections were subjected to HE staining, azocarmine-aniline blue (AZAN) staining, and immunostaining for CD31.

The length of the neo-epithelium was measured using an optical microscope on HE-stained sections. The wound site and the neo-epithelium have no hair follicles, while the surrounding tissue has hair follicles, therefore the wound site was identified using the hair follicles as an indicator, and the layer of epithelial cells formed continuously on top of the wound site (stained dark blue with hematoxylin) was determined to be the neo-epithelium.

The area of newly formed dermis-like tissue on the muscle layer of the wound site was measured using an optical microscope on AZAN-stained sections. The fibrous connective tissue in the granulation tissue is stained light blue with aniline blue and can be distinguished from the dermis at the edge of the wound, which is stained dark blue, therefore the light blue stained portion was determined to be neo-dermis-like tissue, and its area was measured by BZ-X800 analysis software (Keyence Corporation).

The total area of neo-capillaries per unit area of the neo-dermis-like tissue was measured on sections from immunostaining (DAB staining) with an anti-CD31 antibody performed by a conventional method. A threshold was set for the brown color of the DAB staining, and the area with a color density equal to or greater than this threshold was measured using BZ-X800 analysis software (Keyence Corporation). Then, the area where the tubular structure of blood vessels was visible was measured, the total of this area was calculated as the area of neo-capillaries, and the result of dividing by the granulation area determined by AZAN staining was taken as the neo-capillary area per unit granulation area.

### (3) Statistical Analysis

Statistical significance was evaluated using the Tukey-Kramer multiple comparison method. All data are expressed as mean ± standard deviation, and P<0.05 was considered statistically significant.

### (4) Results

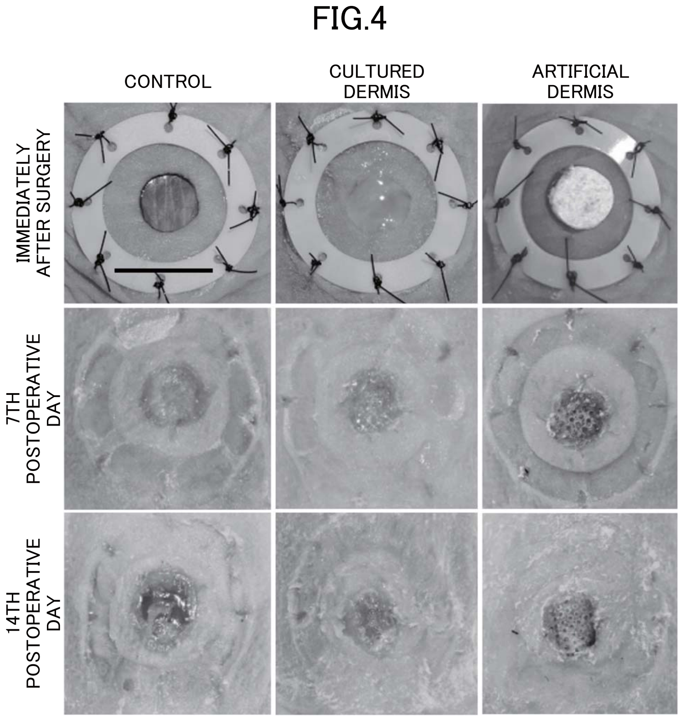

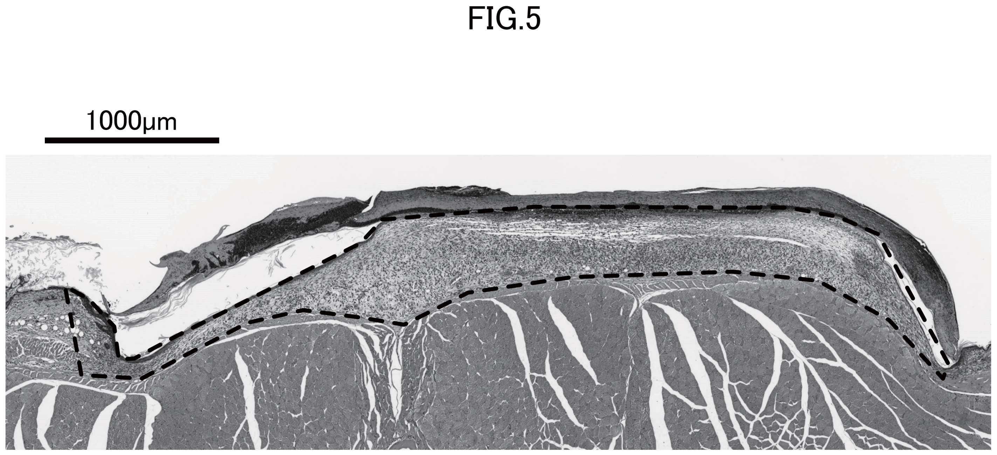

Fig. 4 shows photographs of the wound site immediately after surgery, on the 7th postoperative day, and on the 14th postoperative day. The wounds were covered with eschar. No clear signs of infection were observed, and it was confirmed that the procedure was performed appropriately. Fig. 5 shows a photograph of a section of the wound site stained with HE. Engraftment of the transplanted cultured dermis (the portion enclosed by a dashed line) was confirmed.

Table 2 shows the length of the neo-epithelium at the wound site on the 7th and 14th postoperative days. In the cultured dermis group, on the 7th and 14th postoperative days, the neo-epithelium was formed longer compared to the control group and the commercially available artificial dermis group.

**Table 2: Length of Neo-epithelium (mean ± standard deviation, mm)**

| Test Group | 7th Postoperative Day | 14th Postoperative Day |
|---|---|---|
| Control Group | 1.977±0.728 | 3.212±1.435 |
| Cultured Dermis Group | 2.723±1.155 | 5.502±0.353* |
| Commercially Available Artificial Dermis Group | 2.062±0.411 | 3.590±0.865 |

| | | |
|---|---|---|
| * p<0.05 | | |

When comparing the area of neo-dermis-like tissue at the wound site on the 7th and 14th postoperative days, although there was no significant difference among the groups, in the cultured dermis group, after the 7th postoperative day, there was a tendency for neo-dermis-like tissue to be formed over a wider area compared to the control group and the commercially available artificial dermis group.

Table 3 shows the area of neo-capillaries at the wound site on the 7th and 14th postoperative days. In the cultured dermis group, on the 14th postoperative day, more capillaries were formed compared to the control group and the artificial dermis group.

**Table 3: Area of Neo-capillaries mean ± standard deviation, mm²)**

| Test Group | 7th Postoperative Day | 14th Postoperative Day |
|---|---|---|
| Control Group | 7.489±3.555 | 2.906±0.656 |
| Cultured Dermis Group | 7.035±2.173 | 5.784±1.940* |
| Commercially Available Artificial Dermis Group | 6.355±1.214 | 2.795±0.830 |

| | | |
|---|---|---|
| * p<0.05 | | |

### (5) Discussion

The three-dimensional cell structure produced according to the method of the present invention engrafted at the wound site and functioned as a cultured dermis. Fibroblasts are known to produce cytokines that promote angiogenesis, therefore, it is thought that by transplanting the three-dimensional cell structure as a cultured dermis, vascular epithelial cells, fibroblasts, and the like entered its interior, and the formation of neo-capillaries and the formation of neo-epithelial cells were promoted.

### [Test Example 4] Co-culture with Epidermal Cell Sheet

A three-dimensional cell structure produced by culturing for 4 months by the method described in Test Example 1 was cut out with a 6 mm diameter biopsy punch (Kai Industries Co., Ltd.), and was divided horizontally into two upper and lower three-dimensional cell structures using forceps for use as cultured dermis for transplantation. A 24-well cell insert was set in a 24-well plate, the cultured dermis for transplantation was placed into the cell insert such that the portion that was the outermost layer of the three-dimensional cell structure faced up, an epidermal cell sheet (JACE^{(R)}, Japan Tissue Engineering Co., Ltd.) cut out to the same size was stacked on top of it, 0.9 mL of fibroblast serum-free medium (FKCM) supplemented with 10% fetal bovine serum was added to each well, and air-liquid interface culture was performed under conditions of 37°C and 5% CO₂.

After 1 day of co-culture, the co-culture was collected, and it was transplanted to a skin defect site in the same manner as in Test Example 3. When the wound healing state was confirmed on the 7th postoperative day, engraftment of the transplanted co-culture and formation of neo-epithelium were confirmed.

### [Test Example 5] Simple Culture of Large Three-dimensional Cell Structure

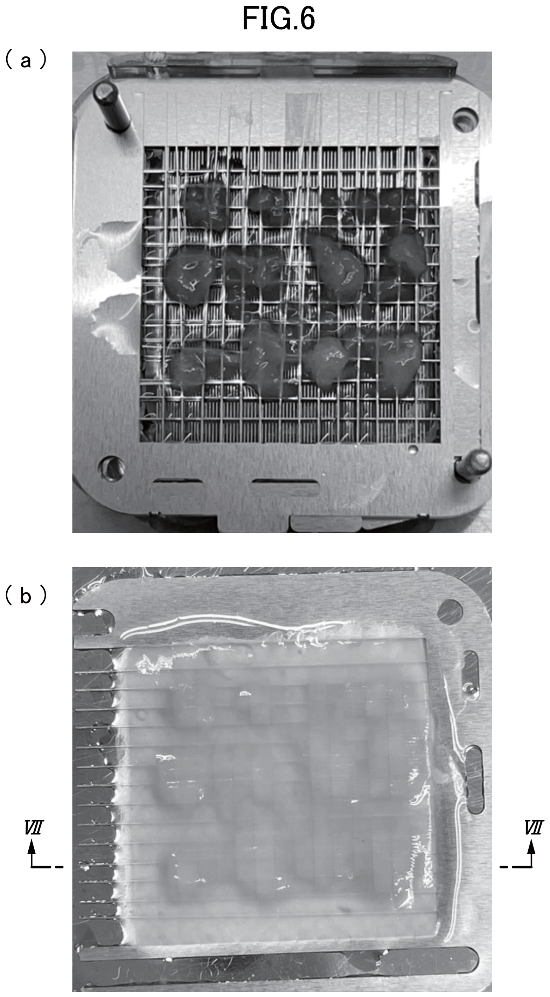

NHDF in spheroid form was prepared in the same manner as in (2) of Test Example 1, and those that had become large aggregates (with a diameter of 1000 µm or more) were selected. A Net Mold NM14-2 (Tissue By Net Inc.) was prepared as a culture chamber, the interval of its stainless steel wires was adjusted to 1 mm both vertically and horizontally, and spheroids were placed at equal intervals on the cavity (Fig. 6a). The top surface of the culture chamber was set by a conventional method to push the spheroids into the internal space of the cavity, and they were cultured in the same manner as in (3) of Test Example 1.

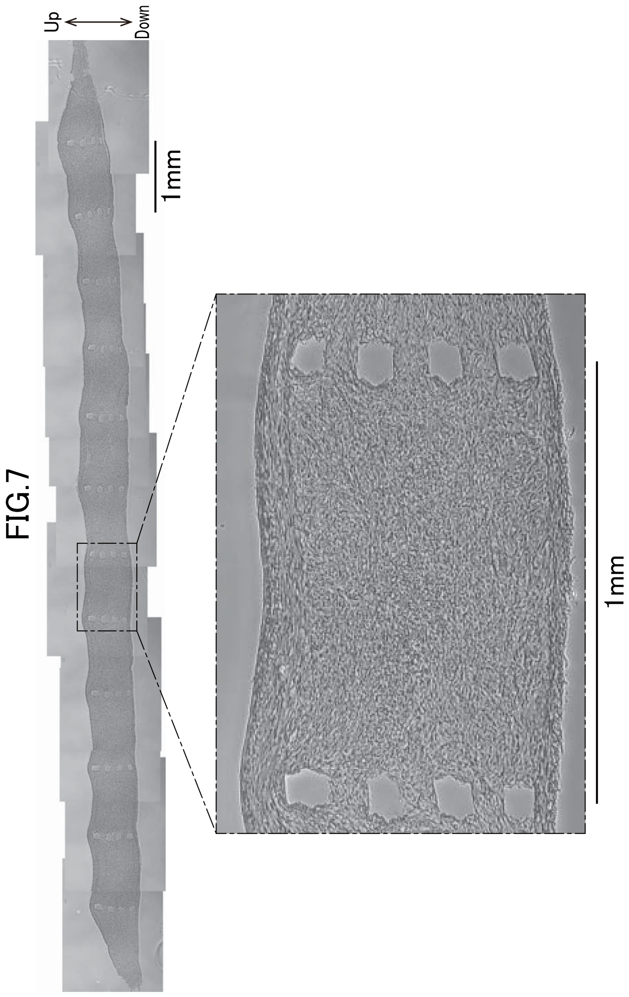

After culturing for one month, an external appearance photograph when the upper and lower nets of the Net Mold were removed leaving only one intermediate net is shown in Fig. 6b. The original cells in a spheroid state grew beyond the cavity portion where they were placed at the beginning of culture, spreading and proliferating throughout the entire culture chamber, and formed a three-dimensional cell structure. When a cross-section of this three-dimensional cell structure was stained, as shown in Fig. 7, it was found that the cells had reached the edges of the three-dimensional cell structure (the large voids in Fig. 7 are traces of the wires that partitioned the cavity). Also, looking at the partially enlarged photograph in Fig. 7, even in the three-dimensional cell structure produced by the method of this test example, it was confirmed that outside the central portion where the spheroids existed at the start of culture, an intermediate layer with a relatively low cell density but constituting a tissue via an extracellular matrix, and an outermost layer containing a dense multilayer structure on the surface of the three-dimensional cell structure were formed; that is, it was confirmed that a structure similar to a living body's cell tissue structure (e.g., dermal structure) was formed. Therefore, by adjusting the usage of the culture chamber, a three-dimensional cell structure of a desired size can be produced.

From the above, it was found that by growing a three-dimensional cell structure until it overflows from the cavity of a culture chamber, a three-dimensional cell structure having a strength capable of being sutured can be produced. Such a three-dimensional cell structure is useful as a biological transplant such as a cultured dermis.

### Reference Signs List

1 wire
11 internal space of the cavity of the culture chamber
2 original cells
3 culture medium
4 three-dimensional cell structure formed in the internal space of the cavity
41 three-dimensional cell structure that has grown until it overflows from the cavity
411 outermost layer of the three-dimensional cell structure
412 intermediate layer of the three-dimensional cell structure
413 central portion of the three-dimensional cell structure

## Claims

1. A method for producing a three-dimensional cell structure, comprising:
(1) a step of preparing original cells having proliferative ability, and a culture chamber that includes a cavity having an internal space enclosed by a bottom surface, a top surface, and side surfaces, and that is formed such that a culture medium can circulate within the internal space;
(2) a step of supplying the original cells to the culture chamber;
(3) a step of holding the entire culture chamber in a culture medium; and
(4) a step of culturing the original cells to form a three-dimensional cell structure that is configured by the original cells, cultured cells derived therefrom, and an extracellular matrix produced during culture becoming integrated, and that has grown to outside the cavity.

2. The method according to claim 1, wherein the original cells include fibroblasts and/or cells having fibroblast differentiation potential.

3. The method according to claim 1, wherein the original cells are in the form of spheroids.

4. The method according to claim 1, wherein step 2 includes a step of filling the internal space of the cavity with the original cells.

5. The method according to claim 1, wherein the original cells are cultured until the breaking force of the three-dimensional cell structure becomes 0.08 N or more.

6. The method according to claim 1, further comprising a step of collecting a portion of the three-dimensional cell structure that has overflowed to the outside of the cavity.

7. The method according to claim 1, wherein the three-dimensional cell structure includes a cultured dermis.

8. The method according to claim 1, wherein the bottom surface and/or the top surface of the cavity has a comb-like shape or a net-like shape.

9. The method according to claim 1, wherein the side surfaces of the cavity are formed by laminating a plurality of frames.

10. A method for producing artificial skin, comprising a step of laminating a three-dimensional cell structure produced by the method according to any one of claims 1 to 9 with a member for an epidermis.

11. A three-dimensional cell structure that includes original cells used at the start of culture therein, and that is configured by the original cells, cultured cells derived therefrom, and an extracellular matrix produced during culture becoming integrated,
wherein the breaking force is 0.08 N or more.

12. The three-dimensional cell structure according to claim 11, wherein the original cells include fibroblasts and/or cells having fibroblast differentiation potential.

13. The three-dimensional cell structure according to claim 11, including a cultured dermis.

14. Artificial skin, comprising the three-dimensional cell structure according to any one of claims 11 to 13 and a member for an epidermis laminated thereon.
